# EUROPEAN PATENT APPLICATION

(11) **EP 1 952 798 A1**
(43) Date of publication of application: **06.08.2008**
(21) Application number: 08000253.8
(22) Date of filing: 09.01.2008
(51) Int. Cl.: A61K 8/49, A61K 8/67, A61Q 7/00

(54) **Cosmetic composition and method to encourage the re-growth of hair**

(30) Priority: 24.01.2007 IT GE20070009
(71) Applicant: Mulargia, Fabio, 18038 Sanremo (IM) (IT); Baeram, Leila, 18038 Sanremo (IM) (IT)
(72) Inventor: Mulargia, Fabio, 18038 Sanremo (IM) (IT); Baeram, Leila, 18038 Sanremo (IM) (IT)
(74) Representative: Sergio, Stefano

(57) **Abstract**

Cosmetic composition aimed at encouraging the re-growth of hair comprising melatonin in addition to retinol and tocopherol.

## Description

This invention is referred to a cosmetic composition and a method to encourage the re-growth of hair that uses the aforesaid cosmetic composition.

The problem of the early loss of hair, due to natural causes or even possibly caused by the taking of drugs is an aesthetic problem that is widely felt. Many specialised companies in the sector have produced products for this type of problem with results that are less than sensational.

This invention proposes to resolve this problem by creating a cosmetic composition to be used to encourage the re-growth of hair and the corresponding treatment to be carried out in a predetermined period of time.

These aims are reached by the cosmetic composition according to claim 1, and by the method to encourage the re-growth of hair according to claim 3 to which one is referred for brevity.

The cosmetic composition according to this invention preferably includes melatonin in addition to retinol and tocopherol to be taken together for a minimum of 8 days up to a maximum of 18 days.

Melatonin is a natural substance produced by the pineal gland (epiphysis) and is a very important substance to maintain equilibrium in the neuroendocrine/hormonal system. Retinol/tocopherol alpha acetate is substantially the active ingredient of a vitamin preparation based on vitamin A and vitamin E. This composition may be taken by the user in, for example, tablets or phials or other means for cycles of at least 15 days, preferably for 30 days, in which the daily dosage includes 30,000 UI of retinol and 70 mg of tocopherol acetate.

The method for the re-growth of hair, besides the main application of the cosmetic composition described above, presents preferably also the taking of:
- Polydeoxyribonucleotide (PDRN) preferably to be taken in quantities of 0.75 mg, which is obtained by hot extraction from human placentas and is commercialised in the form of phials (PLACENTEX) of 3 ml or creams with a cicatrizing or regenerative function. This dosage is advantageously advised daily from 3 to 6 ml. In the case in which the treatment with the cosmetic composition is of 30 days, said quantity may be preferably taken from the first to the tenth day of the treatment and from the twenty-first to the thirtieth day of treatment, In general, said product may be used in the first 6-8 days and the last 12-18 days of treatment;
- a quantity of thiamine hydrochloride (Vitamin B₁) for instance in the quantity of 38 mg, pyridoxine hydrochloride (Vitamin B₆) for instance the quantity of 200 mg, and cyanocobalamin (Vitamin B₁₂) for instance in the quantity of 1000 mg. Said quantities may be taken daily in lyphophilised phials according to the quantities indicated above, which are commercialised with the product BENEXOL. In the case in which the treatment with the cosmetic composition is of 30 days, said quantity may be taken preferably from the eleventh to the twentieth day, or alternatively from the sixth to the eighteenth day of treatment.
In addition to said cosmetic composition and to said treatment for the re-growth of hair it is advantageous to apply a mask to the scalp including powdered clay, olive oil, 5-10 drops of Ylang-Ylang plus a zinc oxide cream for instance 20-40%. This mask may be advantageously used for at least 2-4 times a week.

Therefore the treatment varies from one subject to another and the results can be seen a couple of months after the end of the treatment.

## Claims

1. Cosmetic composition aimed at encouraging the re-growth of hair **characterised by** the fact of including melatonin in addition to retinol and tocopherol.

2. Composition according to claim 1, in which said retinol and tocopherol acetate are to be taken in quantities of respectively 30,000 UI of retinol and 70 mg of tocopherol acetate.

3. Method to encourage the re-growth of hair **characterised by** the fact of including the following phase of taking for a predetermined period of at least fifteen days the cosmetic composition according to claim 1, in a daily quantity of 30,000 UI of retinol and 70 mg of tocopherol acetate .

4. Method according to claim 3, further including the phase of taking polydeoxyribonucleotide (PDRN), which may be taken daily preferably in quantities of 0.75 mg, in an initial portion of said predetermined period and in a later final portion.

5. Method according to claim 4, in which if said predetermined period is of thirty days said initial portion may be of 8-10 days and said final portion of 10-12 days.

6. Method according to claim 3, further including the phase of taking thiamine hydrochloride (Vitamin B₁), for instance in the quantity of 38 mg, pyridoxine hydrochloride (Vitamin B₆) for instance the quantity of 200 mg, and cyanocobalamin (Vitamin B₁₂) for instance in the quantity of 1000 mg on a daily basis in an intermediate portion of said predetermined period.

7. Method according to claim 6, in which if said predetermined period is of thirty days said intermediate portion may be of 8-12 days.

8. Method according to claim 3, further including the phase of application on the scalp of a mask containing powdered clay, olive oil, 5-10 drops of Ylang-Ylang plus a zinc oxide cream, for instance of 20-40%, for at least 2-4 times a week for the entire predetermined period.
